(19) Europäisches Patentamt
European Patent Office
Office européen des brevets

(11) **EP 3 156 071 A1**

(12) **EUROPEAN PATENT APPLICATION**
published in accordance with Art. 153(4) EPC

(43) Date of publication:
**19.04.2017 Bulletin 2017/16**

(21) Application number: **15807343.7**

(22) Date of filing: **04.06.2015**

(51) Int Cl.:
*A61K 39/395* (2006.01)    *A61K 9/08* (2006.01)
*A61K 47/02* (2006.01)    *A61K 47/22* (2006.01)
*A61P 1/04* (2006.01)    *A61P 17/06* (2006.01)
*A61P 19/02* (2006.01)    *A61P 29/00* (2006.01)

(86) International application number:
**PCT/JP2015/066136**

(87) International publication number:
**WO 2015/190378 (17.12.2015 Gazette 2015/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **10.06.2014 JP 2014119306**

(71) Applicants:
• **Meiji Seika Pharma Co., Ltd.**
**Tokyo 104-8002 (JP)**

• **Dong-A Socio Holdings Co., Ltd.**
**Seoul 130-823 (KR)**

(72) Inventors:
• **KAYA, Arpansiree**
**Odawara-shi**
**Kanagawa 250-0852 (JP)**
• **FUJITA, Michinari**
**Odawara-shi**
**Kanagawa 250-0852 (JP)**

(74) Representative: **Campbell, Patrick John Henry**
**J A Kemp**
**14 South Square**
**Gray's Inn**
**London WC1R 5JJ (GB)**

(54) **STABLE AQUEOUS ADALIMUMAB PREPARATION**

(57) The present invention provides a stable aqueous adalimumab formulation by incorporating a histidine buffer solution and a phosphate buffer solution in combination. The incorporation of the histidine buffer solution and the phosphate buffer solution makes it possible to improve the stability of adalimumab.

EP 3 156 071 A1

**Description**

[Technical Field]

The present invention relates to a pharmaceutical composition comprising adalimumab and a method for producing the pharmaceutical composition.

[Background Art]

Because of the complicated structures of polypeptides, pharmaceuticals containing polypeptides as active ingredients (polypeptidepharmaceuticals) are susceptible to various chemical changes and physical changes such as aggregation during production processes or storage. It is generally known that these changes not only lower the pharmacological action, but also exert great influences on the safety such as antigenicity.

For preparing a polypeptide drug, a pharmaceutical formulation is necessary which enables stable storage of a polypeptide used as an active ingredient (Nat. Rev. Drug. Discov., 2005, 4(4), 298-306 (NPL 1)). In general, degradations of polypeptides include the following two: degradation associated with chemical modification such as hydrolysis, deamidation, or methionine oxidation (Int. J. Pharm., 1999, 185, 129-188 (NPL 2)); and degradation associated with physical association such as aggregation, cleavage, or denaturation (Int. J. Pharm., 2005, 289, 1-30 (NPL 3)). For use as a pharmaceutical, such degradations during a storage period are required to be reduced as much as possible. Methods for this reduction include those based on selection of the dosage form; optimization of the solution pH; optimization of the types and concentrations of the buffer solution, salt, and stabilizer; and the like.

Adalimumab is a polypeptide (antibody) used for treatment of a connective tissue disease and/or an autoimmune disease such as rheumatoid arthritis, and adalimumab in the form of an aqueous formulation is considered to undergo physical and chemical changes due to temperature, humidity, light, or the like and consequently undergo decrease in activity, like many other polypeptides.

Meanwhile, since most polypeptide pharmaceuticals are used in the form of injections or infusions, aqueous formulations usable as liquid formulations are excellent in convenience. To avoid denaturation, aggregation, and other changes of a polypeptide in an aqueous formulation, the aqueous formulation has to contain additives such as a stabilizer.

For polypeptide stabilization, saccharides, amino acids, water-soluble polymers, nonionic surfactants, buffering agents, and the like have been used as the additives. For example, a succinate buffer solution with a pH of 6.25, a histidine buffer solution with a pH of 6.25, and an acetate-arginine buffer solution with a pH of 6.5 were used in Examples of International Publication No. WO2013/186230 (PTL 1). Meanwhile, a succinate buffer solution, a histidine buffer solution, and a tartrate buffer solution (each with a pH of 5.2) were used in Examples of International Publication No. WO2014/039903 (PTL 2).

Adalimumab is an active ingredient in a pharmaceutical composition marketed as "HUMIRA (registered trademark)" by Abbott, and is a human anti-human TNFα monoclonal antibody used for treatment of autoimmune diseases such as rheumatoid arthritis, psoriasis, and Crohn's disease. "HUMIRA (registered trademark)" is an aqueous liquid formulation in which approximately 50 mg/mL adalimumab, 6.17 mg/mL NaCl, 12 mg/mL mannitol, and 1 mg/mL polysorbate 80 are contained in a 14.1 mM phosphate-4.2 mM citrate buffer solution, and which has a pH of approximately 5.2. For this reason, adalimumab is generally considered to be stable at a pH of 5.2.

It is known that citric acid incorporated in injections causes pain (Am J Kidne y Dis., 1993, Oct 22(4), 553-556 (NPL 4), Clin Nephrol, 1998, Jan 49(1), 41-44 (NPL 5)). "HUMIRA (registered trademark)" seems to cause pain at administration, because it contains citric acid and also has a relatively low pH of approximately 5.2.

[Citation List]

[Patent Literature]

[PTL 1] International Publication No. WO2013/186230
[PTL 2] International Publication No. WO2014/039903

[Non Patent Literature]

[NPL 1] Nat. Rev. Drug. Discov., 2005, 4(4), 298-306

[NPL 2] Int. J. Pharm., 1999, 185, 129-188
[NPL 3] Int. J. Pharm., 2005, 289, 1-30
[NPL 4] Am J Kidney Dis., 1993, Oct 22(4), 553-556
[NPL 5] Clin Nephrol, 1998, Jan 49(1), 41-44

[Summary of Invention]

[Technical Problem]

An object of the present invention is to provide a stable and highly convenient aqueous adalimumab formulation.

[Solution to Problem]

To achieve the above-described object, the present inventors have conducted intensive study, and consequently reached the present invention. Specifically, the present inventors have found that an aqueous adalimumab formulation better in stability than conventional ones can be obtained by using a combination of specific buffer solutions among various buffer solutions, and adjusting the pH within a specific range.
More specifically, the present invention provides the following:

(1) A pharmaceutical composition, which is an aqueous liquid formulation, comprising:

adalimumab; and
a histidine buffer solution and a phosphate buffer solution as a buffer solution, wherein
the pharmaceutical composition has a pH of 5.5 to 6.1.

(2) The pharmaceutical composition according to (1), further comprising at least one additive selected from buffering agents, excipients, and tonicity adjusting agents.
(3) The pharmaceutical composition according to (1) or (2), which has a histidine concentration of 5 mM to 50 mM.
(4) The pharmaceutical composition according to any one of (1) to (3), which has a phosphate concentration of 1 mM to 20 mM.
(5) A pharmaceutical composition, which is a frozen formulation obtained by freezing the pharmaceutical composition according to any one of (1) to (4).
(6) A pharmaceutical formulation, comprising the pharmaceutical composition according to any one of (1) to (4) stored in a prefilled sterile syringe.
(7) A kit preparation, comprising:

at least one of the pharmaceutical composition according to any one of (1) to (5) and the pharmaceutical formulation according to (6); and
a specification manual of the pharmaceutical composition.

[Advantageous Effects of Invention]

The configurations of the present invention make it possible to improve the stability of adalimumab in the aqueous formulation. Consequently, improvement of the stability of the pharmaceutical composition comprising adalimumab can be expected, and prevention of decrease in the pharmacological action can be expected. Moreover, improvements in safety such as decrease in antigenicity can also be expected. In addition, it is expected that the pain at injection can be reduced, and the load on the patient can be reduced.

[Brief Description of Drawings]

[Fig. 1] Fig. 1 is a graph showing the monomer content in each aqueous adalimumab formulation after storage at 40°C for one month.
[Fig. 2] Fig. 2 is a graph showing the acidic product content in each aqueous adalimumab formulation after storage at 40°C for one month.
[Fig. 3] Fig. 3 is a graph showing the change ratio of aggregates in some aqueous adalimumab formulations after freeze-thawing with respect to that before the freeze-thawing.

[Description of Embodiments]

In the present invention, an aqueous formulation (hereinafter, sometimes referred to as "aqueous adalimumab formulation") comprising adalimumab, which is a polypeptide, is stabilized by incorporating a histidine buffer solution and a phosphate buffer solution as a buffer solution.

In the present invention, the term "pharmaceutical composition" is understood to refer to a formulation (pharmaceutical formulation) which comprises a peptide and which is prepared to be suitable for injection and/or administration to a patient who needs to be treated. The pharmaceutical composition may comprise a buffering agent, a tonicity adjusting agent, an excipient, and the like, in addition to the buffer solution.

In the present invention, the term "aqueous formulation" is understood to refer to a formulation which is the above-described pharmaceutical formulation and which uses water as a solvent, and the term "aqueous adalimumab formulation" is understood to refer to a formulation comprising at least water and adalimumab. These formulations may comprise additives such as a buffering agent, a tonicity adjusting agent, and an excipient, in addition to the buffer solution. In addition, in the present invention, the term "aqueous liquid formulation" is understood to refer to a liquid formulation which is in the form of the above-described aqueous formulation and which uses water as a solvent, and the term "frozen formulation" is understood to refer to a formulation obtained by freezing the above-described aqueous liquid formulation.

In the present invention, the "aqueous liquid formulation" is preferably used as an injection or an infusion, and preferably stored in a syringe such as a prefilled sterile syringe or in an infusion pack. In addition, the "frozen formulation" is preferably used as an injection or an infusion after being thawed.

In the present invention, the term "kit preparation" is understood to refer to a preparation comprising the above-described pharmaceutical composition and a specification manual of the pharmaceutical composition, and the pharmaceutical composition may be the above-described aqueous liquid formulation or the above-described frozen formulation.

The pH of the pharmaceutical composition of the present invention is 5.5 to 6.1. The present inventors have found that when a histidine buffer solution and a phosphate buffer solution are used in combination, a better stability than that of "HUMIRA (registered trademark)," which is a conventional aqueous adalimumab formulation, is exhibited, and have found that when, in addition to this, the pH is adjusted in a range of 5.5 to 6.1, for example, the content of the adalimumab active main body is maintained during storage and/or the increase of components other than the active main body is suppressed, and thus a further stability of adalimumab is exhibited.

In the present invention, the "buffer solution" is a solution whose pH is maintained within an acceptable range by an action of acid-base conjugate components therein. Examples of the buffer solution include a histidine buffer solution, a phosphate buffer solution, an acetate buffer solution, a succinate buffer solution, a citrate buffer solution, organic acid buffer solutions, and the like. In the present invention, it is possible not only to use a combination of a histidine buffer solution and a phosphate buffer solution as the buffer solution, but also to further combine this combination with another buffer solution, such as, for example, an acetate buffer solution.

In the present invention, the "histidine buffer solution" is a buffer solution prepared from at least two selected from the group consisting of histidine, histidine salts, and hydrates of histidine salts (cases of two histidine salts and cases of two hydrates of histidine salts are also included), and examples of the histidine salts include histidine hydrochloride and the like. Meanwhile, the "phosphate buffer solution" in the present invention is a buffer solution prepared from at least two selected from the group consisting of phosphoric acid, phosphoric acid salts, and hydrates of phosphoric acid salts (cases of two phosphoric acid salts and cases of two hydrates of phosphoric acid salts are also included), and examples of the phosphoric acid salts include sodium phosphate, potassium phosphate, and the like.

In the present invention, the "buffering agent" is a compound having a buffering effect of maintaining the pH in an acceptable range. In addition to histidine, histidine hydrochloride, phosphoric acid, sodium phosphate, and potassium phosphate which may be contained in the histidine buffer solution and the phosphate buffer solution, the buffering agent is preferably at least one selected from the group consisting of sodium citrate, potassium citrate, citric acid, maleic acid, sodium acetate, potassium acetate, ammonium acetate, acetic acid, succinic acid, sodium succinate, tartaric acid, sodium tartrate, lactic acid, sodium lactate, malic acid, boric acid, ascorbic acid, glutamic acid, sodium glutamate, arginine, arginine hydrochloride, tris-(hydroxymethyl)-aminomethane (tris), and diethanolamine, of which histidine, histidine hydrochloride, and sodium phosphate are more preferable.

In the present invention, the tonicity adjusting agent is preferably at least one tonicity adjusting agent selected from the group consisting of sodium chloride, potassium chloride, sodium citrate, sucrose, glucose, mannitol, sorbitol, xylitol, arginine, cysteine, and histidine, and is more preferably sodium chloride.

In the present invention, the excipient is preferably at least one excipient selected from the group consisting of sorbitol, mannitol, sucrose, xylitol, trehalose, glucose, lactose, glycerol, maltose, inositol, bovine serum albumin (BSA), dextran, PVA, hydroxypropyl methyl cellulose (HPMC), polyethylenimine, gelatin, polyvinylpyrrolidone (PVP), hydroxyethylcellulose (HEC), polyethylene glycol, ethylene glycol, dimethyl sulfoxide (DMSO), dimethylformamide (DMF), proline, L-serine, sodium glutamate, potassium glutamate, alanine, lysine hydrochloride, sarcosine, γ-aminobutyric acid, polysorbate 20, polysorbate 80, SDS, polyoxyethylene copolymers, sodium acetate, ammonium sulfate, magnesium sulfate, sodiumsulfate, trimethylamine N-oxide, betaine, zinc ions, copper ions, calcium ions, manganese ions, magnesium ions, CHAPS, sucrose monolaurate, and 2-O-β-mannoglycerate. Sorbitol, mannitol, sucrose, and xylitol are more preferable.

In the present invention, "adalimumab" refers to a protein which is a human anti-human TNFα monoclonal antibody contained as the active ingredient in a pharmaceutical composition marketed as "HUMIRA (registered trademark)," or a protein pharmaceutically equivalent thereto. In addition, a "monomer" of adalimumab in the present invention refers to an active-form protein of approximately 148 kDa. Moreover, in the present invention, the term "active main body" refers to a monomer of adalimumab, and is understood to refer to a monomer which can be identified by size exclusion chromatography (SEC).

The pharmaceutical composition of the present invention comprises: adalimumab; water; and histidine and phosphate incorporated as the buffer solution (and, if necessary, as the buffering agents). A histidine concentration in the pharmaceutical composition of the present invention is not particularly limited. A lower limit value of the histidine concentration is preferably 5 mM, more preferably 10 mM, further preferably 15 mM, and particularly preferably 20 mM, whereas an upper limit value thereof is preferably 50 mM, more preferably 30 mM, further preferably 25 mM, and particularly preferably 20 mM. In addition, the histidine concentration in the pharmaceutical composition of the present invention is preferably 5 mM to 50 mM, more preferably 10 mM to 30 mM, and further preferably 15 mM to 25 mM. Likewise, a phosphate concentration is not particularly limited. A lower limit value of the phosphate concentration is preferably 1 mM, more preferably 3 mM, and further preferably 5 mM, whereas an upper limit value thereof is preferably 20 mM, more preferably 10 mM, and further preferably 5 mM. In addition, the phosphate concentration in the pharmaceutical composition of the present invention is preferably 1 mM to 20 mM, and more preferably 3 mM to 10 mM.

In addition, the pharmaceutical composition of the present invention preferably comprises substantially no glycine. In the present invention, the expression "comprises substantially no glycine" means that the glycine content in the pharmaceutical composition is 6 mM or less. In the present invention, the stability of adalimumab in the aqueous formulation is excellent, even though no glycine is contained.

[Examples]

In the following Examples, the present invention will be described in detail; however, the scope of the present invention is not limited to these Examples.

(Preparation 1 of Aqueous Adalimumab Formulations)

Samples (aqueous adalimumab formulations) in which various buffer solutions were incorporated were prepared (Table 1). Each of these samples was prepared as an aqueous solution which contained 50 mg/mL adalimumab, 6.16 mg/mL NaCl, 12 mg/mL mannitol, 1 mg/mL polysorbate 80, and the corresponding one of the buffer solutions shown in Table 1 and which had a ph of 5.2 to 6.0. In addition, the phosphate buffer solution was prepared from sodium dihydrogen phosphate dihydrate and disodium hydrogen phosphate dodecahydrate, the histidine buffer solution was prepared from histidine and histidine hydrochloride monohydrate, and the citrate buffer solution was prepared from citric acid monohydrate and trisodium citrate dihydrate. These buffer solutions were incorporated in the combinations shown in Table 1 with the concentrations (final concentrations) of the acid components (phosphate, citrate, and histidine) derived from these buffer solutions in the aqueous adalimumab formulations being as shown in Table 1.

[Table 1]

| Formula No. | Concentrations (buffer liquid composition) | pH |
|---|---|---|
| F1 (Comp. Ex. 1) | 14.1 mM phosphate, 4.2 mM citrate | 5.2 |
| | (phosphate buffer solution·citrate buffer solution) | |
| F2 (Comp. Ex. 2) | 25 mM histidine | 5.2 |
| | (histidine buffer solution) | |
| F3 (Comp. Ex. 3) | 25 mM histidine | 6.0 |
| | (histidine buffer solution) | |
| F4 (Ex. 1) | 20 mM histidine, 5 mM phosphate | 5.5 |
| | (histidine buffer solution·phosphate buffer solution) | |

(continued)

| Formula No. | Concentrations (buffer liquid composition) | pH |
|---|---|---|
| F5 (Ex. 2) | 20 mM histidine, 5 mM phosphate | 5.8 |
| | (histidine buffer solution · phosphate buffer solution) | |
| F6 (Ex. 3) | 20 mM histidine, 5 mM phosphate | 6.0 |
| | (histidine buffer solution · phosphate buffer solution) | |

After storage at 40°C, each of the prepared samples was analyzed by size exclusion chromatography (SEC) and ion-exchange chromatography (IEC). The SEC is a technique by which high-molecular weight species (including aggregates), monomer (active form), and low-molecular weight species (including fragments and degradation products) in a sample are separated from each other by pores of a column packing material. For the evaluation, the monomer content (% Monomer) in each sample was measured and evaluated by using this method.

The IEC is a technique by which biological molecules are separated from each other according to characteristics of charges exposed on the protein surface. In the present invention, the content (% Acidic) of the acidic product (deamidation product) formed by deamidation of the active main body of adalimumab in each sample was measured and evaluated by using cation-exchange chromatography.

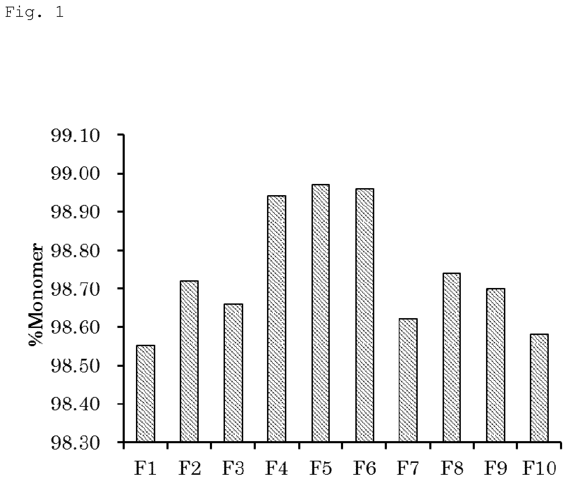

The results of the analysis of the samples by SEC after the storage were as follows. Specifically, after storage at 40°C for one month, a larger amount of the monomer remained in each of Formulae F4 to F6 which contained the histidine buffer solution and the phosphate buffer solution and which had pHs in the range of 5.5 to 6.1 than in Formulae F1 to 3. The % Monomer content of Formulae F1, F2, and F3 were 98.55, 98.72, and 98.66, respectively, whereas the % Monomer content of Formulae F4, F5, and F6 were 98.94, 98.97, and 98.96, respectively, which were remarkably higher than those of Formulae F1, F2, and F3. Hence, Formulae F4 to F6 were formulations excellent in stability (Fig. 1).

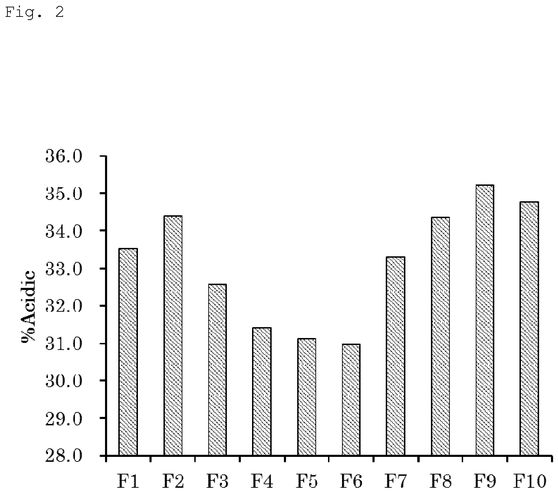

The results of the analysis of the samples by IEC after the storage were as follows. Specifically, after storage at 40°C for one month, the acidic product content was lower in each of Formulae F4 to F6 which contained the histidine buffer solution and the phosphate buffer solution and which had pHs in the range of 5.5 to 6.1 than in Formulae F1 to 3. Accordingly, it was been found that the formation of the deamidation product was suppressed in each of Formulae F4 to F6. The % Acidic content of Formulae F1, F2, and F3 were 33.5, 34.4, and 32.6, respectively, whereas the % Acidic content of Formulae F4, F5, and F6 were as low as 31.4, 31.1, and 31.0, respectively. Hence, Formulae F4 to F6 were formulations excellent in stability (Fig. 2).

In addition, as a freeze-thaw test on Formulae F4 to F6, a freeze-thaw cycle (Freeze-thaw cycle) in which the sample was frozen at -20°C and thawed at room temperature was repeated up to five times, and the aggregate content in each sample was measured by SEC after every cycle. Then, the change ratio (% Change in Aggregate) of the aggregate content with respect to that before the freeze-thawing (that of the sample subjected to no freeze-thaw cycle) was determined by the following formula:

```
change ratio of aggregate content = {[aggregate
content (%) in sample after each freeze-thaw
cycle]/[aggregate content (%) in sample subjected to no
freeze-thaw cycle]} × 100.
```

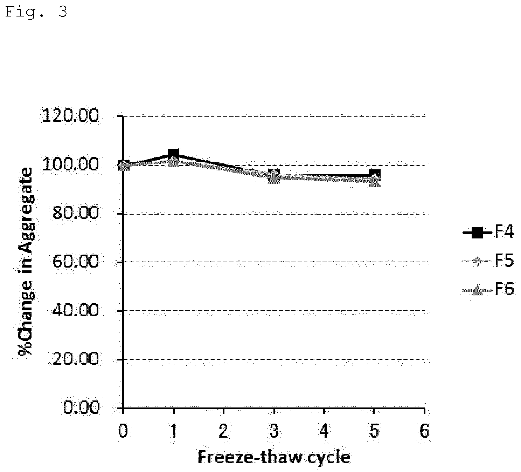

Thus, the stability against freeze-thawing was evaluated. The results showed no apparent increase in aggregates due to the freeze-thawing in each of Formulae F4 to F6, and Formulae F4 to F6 were extremely stable (Fig. 3).

(Preparation 2 of Aqueous Adalimumab Formulations)

Samples (aqueous adalimumab formulations) in which various buffer solutions were incorporated were prepared (Table 2). Each of these samples was prepared as an aqueous solution which contained 50 mg/mL adalimumab, 6.16 mg/mL NaCl, 12 mg/mL mannitol, 1 mg/mL polysorbate 80, and the corresponding one of the buffer solutions shown in Table 2 and which had a ph of 5.2 to 5.5. Inaddition, the phosphate buffer solution and the histidine buffer solution were prepared in the same manner as in Preparation 1 of Aqueous Adalimumab Formulations described above, and the acetate buffer solution was prepared from acetic acid and sodium acetate trihydrate. These buffer solutions were incorporated in the combinations shown in Table 2 with the concentrations (final concentrations) of the acid components (phosphate, histidine, and acetate) derived from the buffer solutions in the aqueous adalimumab formulations being as shown in Table 2.

[Table 2]

| Formula No. | Concentrations (buffer liquid composition) | pH |
|---|---|---|
| F7 (Comp. Ex. 4) | 25 mM histidine | 5.5 |
| | (histidine buffer solution) | |
| F8 (Comp. Ex. 5) | 20 mM histidine, 5 mM phosphate | 5.2 |
| | (histidine buffer solution ·phosphate buffer solution) | |
| F9 (Comp. Ex. 6) | 20 mM histidine, 5 mM acetate | 5.2 |
| | (histidine buffer solution ·acetate buffer solution) | |
| F10 (Comp. Ex. 7) | 12.5 mM histidine, 12.5 mM acetate | 5.2 |
| | (histidine buffer solution·acetate buffer solution) | |

After storage at 40°C for one month, the prepared samples were analyzed by SEC and IEC in the same manner as in Preparation 1 of Aqueous Adalimumab Formulations. The results are shown in Figs. 1 and 2 together with the results of Formulae F1 to F6. As is apparent from the results shown in Figs. 1 and 2, the amount of monomer remaining in Formula 8 which contained the histidine buffer solution and the phosphate buffer solution and had a pH of 5.2 was equal to or more than that remained in Formula 1 having a pH of 5.2 and corresponding to "HUMIRA (registered trademark)." Meanwhile, a larger amount of monomer remained in each of Formulae F4 to F6 which contained the histidine buffer solution and the phosphate buffer solution and which had a pH in the range of 5.5 to 6.1 than in Formula 8. In addition, the formation of the acidic product was suppressed further sufficiently in each of Formulae F4 to F6. Accordingly, it was found that Formulae F4 to F6 were formulations especially excellent in stability.

[Industrial Applicability]

The present invention demonstrates that the stability of adalimumab is improved by using a histidine buffer solution and a phosphate buffer solution in combination. Moreover, it is conceivable that the pain can be reduced and the load on the patient can be reduced by incorporating no citric acid as an additive and adjusting the pH of the formulation to 5.5 to 6.1. These results make it possible to provide a stable and highly convenient aqueous adalimumab formulation.

**Claims**

1. A pharmaceutical composition, which is an aqueous liquid formulation comprising:

   adalimumab; and
   a histidine buffer solution and a phosphate buffer solution as a buffer solution, wherein
   the pharmaceutical composition has a pH of 5.5 to 6.1.

2. The pharmaceutical composition according to claim 1, further comprising at least one additive selected from buffering agents, excipients, and tonicity adjusting agents.

3. The pharmaceutical composition according to claim 1 or 2, which has a histidine concentration of 5 mM to 50 mM.

4. The pharmaceutical composition according to any one of claims 1 to 3, which has a phosphate concentration of 1 mM to 20 mM.

5. A pharmaceutical composition, which is a frozen formulation obtained by freezing the pharmaceutical composition according to any one of claims 1 to 4.

6. A pharmaceutical formulation, comprising the pharmaceutical composition according to any one of claims 1 to 4 stored in a prefilled sterile syringe.

7. A kit preparation, comprising:

at least one of the pharmaceutical composition according to any one of claims 1 to 5 and the pharmaceutical formulation according to claim 6; and
a specification manual of the pharmaceutical composition.

Fig. 1

Fig. 2

Fig. 3

**INTERNATIONAL SEARCH REPORT**

| International application No. |
| --- |
| PCT/JP2015/066136 |

**A. CLASSIFICATION OF SUBJECT MATTER**
*A61K39/395*(2006.01)i, *A61K9/08*(2006.01)i, *A61K47/02*(2006.01)i, *A61K47/22* (2006.01)i, *A61P1/04*(2006.01)i, *A61P17/06*(2006.01)i, *A61P19/02*(2006.01)i, *A61P29/00*(2006.01)i

According to International Patent Classification (IPC) or to both national classification and IPC

**B. FIELDS SEARCHED**

Minimum documentation searched (classification system followed by classification symbols)
A61K39/395, A61K9/08, A61K47/02, A61K47/22, A61P1/04, A61P17/06, A61P19/02, A61P29/00

Documentation searched other than minimum documentation to the extent that such documents are included in the fields searched
Jitsuyo Shinan Koho        1922-1996    Jitsuyo Shinan Toroku Koho    1996-2015
Kokai Jitsuyo Shinan Koho    1971-2015    Toroku Jitsuyo Shinan Koho    1994-2015

Electronic data base consulted during the international search (name of data base and, where practicable, search terms used)
CAplus/MEDLINE/EMBASE/BIOSIS(STN), JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2012-510468 A  (Abbot Laboratories), 10 May 2012 (10.05.2012), paragraphs [0288] to [0291]; table 15.6; claims 52, 81; paragraph [0166] & US 2010/0172862 A1    & WO 2010/062896 A1 & EP 2350649 A        & TW 201036627 A & KR 10-2011-0096553 A  & CN 102301235 A & RU 2011126338 A | 1-7 |
| Y | JP 2012-526121 A  (Abbott Biotechnology Ltd.), 25 October 2012 (25.10.2012), claims; paragraphs [0003], [0204], [0256] & US 2010/0278822 A1    & WO 2010/129469 A1 & EP 2427211 A        & TW 201043263 A & CA 2760185 A        & CN 102458469 A & KR 10-2012-0038406 A  & RU 2011149327 A | 1-7 |

☐  Further documents are listed in the continuation of Box C.        ☐  See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 30 July 2015 (30.07.15) | 18 August 2015 (18.08.15) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013186230 A
- WO 2014039903 A

**Non-patent literature cited in the description**

- *Nat. Rev. Drug. Discov.,* 2005, vol. 4 (4), 298-306
- *Int. J. Pharm.,* 1999, vol. 185, 129-188
- *Int. J. Pharm.,* 2005, vol. 289, 1-30
- *Am J Kidne y Dis.,* 22 October 1993, vol. 4, 553-556
- *Clin Nephrol,* 04 January 1998, vol. 9 (1), 41-44
- *Am J Kidney Dis.,* 22 October 1993, vol. 4, 553-556